# EUROPEAN PATENT SPECIFICATION

(11) **EP 2 723 890 B1**
(45) Date of publication and mention of the grant of the patent: **09.10.2019**
(21) Application number: 12729153.2
(22) Date of filing: 25.06.2012
(51) Int. Cl.: C12Q 1/6883

(54) **NEW TH17 DIFFERENTIATION MARKERS FOR ACNE AND USES THEREOF**
NEUE TH17-DIFFERENZIERUNGSMARKER FÜR AKNE UND IHRE VERWENDUNG
NOUVEAUX MARQUEURS DE DIFFÉRENCIATION DES TH17 POUR L'ACNÉ ET LEURS UTILISATIONS

(30) Priority: 27.06.2011 US 201161501365 P
(43) Date of publication of application: 30.04.2014
(73) Proprietor: Galderma Research & Development, 06410 Biot (FR)
(72) Inventor: CARLAVAN, Isabelle, F-06130 Grasse (FR)
(74) Representative: Nederlandsch Octrooibureau
(86) International application number: PCT/EP2012/062258
(87) International publication number: WO 2013/000870

(56) References cited:
- WO-A2-02/089791
- WO-A2-2007/077257
- TRIVEDI NISHIT R ET AL: "Gene array expression profiling in acne lesions reveals marked upregulation of genes involved in inflammation and matrix remodeling", JOURNAL OF INVESTIGATIVE DERMATOLOGY, NATURE PUBLISHING GROUP, GB, vol. 126, no. 5, 1 May 2006 (2006-05-01), pages 1071-1079, XP002417785, ISSN: 0022-202X, DOI: 10.1038/SJ.JID.5700213
- GUSTAVO J. MARTINEZ ET AL: "Regulation and Function of Proinflammatory TH17 Cells", ANNALS OF THE NEW YORK ACADEMY OF SCIENCES, vol. 1143, no. 1, 1 November 2008 (2008-11-01), pages 188-211, XP55034259, ISSN: 0077-8923, DOI: 10.1196/annals.1443.021 cited in the application
- YEONSEOK CHUNG ET AL: "Critical Regulation of Early Th17 Cell Differentiation by Interleukin-1 Signaling", IMMUNITY, vol. 30, no. 4, 1 April 2009 (2009-04-01), pages 576-587, XP55034079, ISSN: 1074-7613, DOI: 10.1016/j.immuni.2009.02.007 cited in the application
- LIANG ZHOU ET AL: "IL-6 programs TH-17 cell differentiation by promoting sequential engagement of the IL-21 and IL-23 pathways", NATURE IMMUNOLOGY, vol. 8, no. 9, 20 June 2007 (2007-06-20), pages 967-974, XP55022809, ISSN: 1529-2908, DOI: 10.1038/ni1488 cited in the application
- C. DOE ET AL: "Expression of the T Helper 17-Associated Cytokines IL-17A and IL-17F in Asthma and COPD", CHEST, vol. 138, no. 5, 1 November 2010 (2010-11-01), pages 1140-1147, XP55034071, ISSN: 0012-3692, DOI: 10.1378/chest.09-3058 cited in the application
- MICHAEL N. HEDRICK ET AL: "CCR6 is required for IL-23-induced psoriasis-like inflammation in mice", JOURNAL OF CLINICAL INVESTIGATION, vol. 119, no. 8, 3 August 2009 (2009-08-03), pages 2317-2329, XP55034265, ISSN: 0021-9738, DOI: 10.1172/JCI37378 cited in the application
- JOSHUA D. MILNER ET AL: "Impaired TH17 cell differentiation in subjects with autosomal dominant hyper-IgE syndrome", NATURE, vol. 452, no. 7188, 12 March 2008 (2008-03-12), pages 773-776, XP55034270, ISSN: 0028-0836, DOI: 10.1038/nature06764 cited in the application

## Description

The disclosure is related to a novel characterization process of acne, by identifying for the first time in the inflammatory process the involvement of Th17 cells and to the therapeutic applications targeting the function of Th17 cells in acne.

More specially, the disclosure proposes the use of IE-12Rβ1/IL-23R, CCR6, BATF, AHR, STAT3, IRF4 crucial actors in Th17 cells differentiation as new markers for acne, and their use to diagnose acne, to screen inhibitors of Th17 differentiation, notably in inhibiting IL-12Rβ1/IL-23R, CCR6, BATF, AHR, STAT3,IRF4 and the use of these screened inhibitors in acne treatment.

Acne is the most common skin condition affecting millions of people worldwide. Patients with severe acne frequently face significant psychological and emotional problems due to the scarring associated with the disease. The pathogenesis of acne vulgaris is complex and incompletely understood.

Inflammation is one of the key components of the pathogenesis of acne. An immunological reaction to the gram-positive microbe P. acnes may play a major role in the initiation of the inflammatory reaction (De Young LM, Young JM, Ballaron SJ, Spires DA, Puhvel SM. Intradermal injection of Propionibacterium acnes: a model of inflammation relevant to acne. J Invest Dermatol. 1984 Nov;83(5):394-8, Jappe U, Ingham E, Henwood J, Holland KT. Propionibacterium acnes and inflammation in acne; P. acnes has T-cell mitogenic activity. Br J Dermatol. 2002 Feb;146(2):202-9). Recently published studies also implicate Toll Like receptor 2 (TLR-2) in inflammatory acne (Fathy A, Mohamed RW, Ismael NA, El-Akhras MA. Expression of toll-like receptor 2 on peripheral blood monocytes of patients with inflammatory and noninflammatory acne vulgaris. Egypt J Immunol. 2009; 16(1) :127-34; Nagy I, Pivarcsi A, Koreck A, Széll M, Urban E, Kemény L. Distinct strains of Propionibacterium acnes induce selective human beta-defensin-2 and interleukin-8 expression in human keratinocytes through toll-like receptors. J Invest Dermatol.2005 May;124(5):931-8).

Recent reports demonstrate that the skin expresses various antimicrobial peptides in response to the proliferation of pathogens as part of cutaneous innate immunity (Braff MH, Bardan A, Nizet V, Gallo RL. Cutaneous defense mechanisms by antimicrobial peptides. J Invest Dermatol. 2005 Jul;125(1):9-13; Schröder JM. Epithelial antimicrobial peptides: local innate defense effector molecules]. Ann Dermatol Venereol. 2004 Apr;131(4):411-6; Selsted ME, Ouellette AJ. Mammalian defensins in the antimicrobial immune response. Nat Immunol. 2005 Jun;6(6):551-7). Important amongst this group of antimicrobial agents include members of the human β defensin family and granulysin-derived peptides (Deng et al, 2005; Harder et al, 2004; McInturff et al, 2005). Human β defensin-1 and 2 (HBD-1 and HBD-2) are expressed in the pilosebaceous unit and their expression is upregulated in acne lesions (Chronnell et al, 2001). Recent studies have also discovered that select strains of P. acnes can activate HBD-2 through TLRs further confirming the importance of these peptides in inflammatory acne (Nagy, et al., 2005).

For the first time, the applicant proposes with experimental evidences to target a novel inflammatory process, the Th-17 cells differentiation for treating and/or diagnosing acne. Thus, the disclosure is relating to the use of the mRNA encoding IL-12Rbeta 1/IL-23R, CCR6 and also the corresponding proteins, as markers for acne as well as the use of the mRNA encoding at least one of the transcription factors chosen from BATF, AHR, STAT 3, IRF4, and also the corresponding proteins, as markers for acne. The disclosure is also relating to the use of at least one of the markers of the above proposed markers and/or at least one of the markers chosen from IL-6, IL-17A, IL-17F, IL-21, IL-22, IL-26, TNF alpha, CCL20, as markers for acne. The invention also provides a method for the diagnosis of acne, comprising the following steps:
a) detecting the level of expression of the mRNA encoding at least one of the proposed markers and/or at least one of the markers or the corresponding protein chosen from IL-6, IL-17A, IL-17F, IL-22, CCL20 in a sample taken from an individual,
b) detecting the level of expression of the mRNA encoding at least one of the proposed markers and/or at least one of the markers chosen from IL-6, IL-17A, IL-17F, IL-22 and CCL20 in a sample taken from a healthy individual,
c) comparing the difference in level of expression of the mRNA encoding at least one marker or the corresponding protein and for which the level of expression is significantly higher than the level of expression in the healthy individual;
d) the overexpression of at least one of the markers of step c) being an indicator of acne, thus diagnosing acne.

The invention provides also a method for the diagnosis of acne that can also comprise the following steps:
a) detecting the level of expression of at least one of the proposed markers in a sample taken from an individual,
b) detecting the level of expression of at least one of the proposed markers in a sample taken from a normal individual,
c) comparing the difference in level of expression of at least one marker and for which the level of expression is significantly higher than the level of expression in the healthy individual;
d) the overexpression of at least one of the markers of step c) being an indicator of acne, thus diagnosing acne.

The disclosure provides a method for monitoring the progression or variation of acne, comprising the following steps:
a) taking a biological sample from the individual,
b) analysing the level of expression of at least one of the proposed markers, and/or at least one of the markers chosen from IL-6,IL-17A, IL-17F, IL-22, CCL20 in a sample taken and in which a variation in the expression of at least one of the markers is an indicator of the progression of acne. Progression of acne may be from a predominantly comedonal to a more inflammatory dominated state, it may also mean progression towards specific acne subtypes, like nodulocystic acne or acne conglobata for example. Progression might also occur in the other direction, from a more severe to a less severe form of acne.

The disclosure provides also a method for monitoring the efficacy of a treatment intended for treating acne, comprising the following steps:
a) administering the desired treatment to the individual identified as having one or more of the symptoms of acne,
b) taking a biological sample from the individual,
c) analysing the level of expression of at least one of the proposed markers and/or at least one of the other markers chosen from Il-6, IL-17A, IL-17F, IL-22, CCL20, in which a variation in the expression of at least one of the markers is an indicator of efficacy in the treatment of acne.

The disclosure provides also a in vitro screening method of Th-17 cells differentiation inhibitors, comprising determining the capacity of said candidate to inhibit or down regulate expression and/or the biological function of one of the proposed markers.

More specifically, the disclosure relates to an in vitro screening method of Th17 cells differentiation inhibitors for the identification of drug candidates, comprising the following steps:
a) Collecting at least two biological samples: one mimics the acne lesion, and one mimics the healthy condition;
b) Contacting at least one sample or a mixture of samples with one or more drug candidates to be tested;
c) Detecting the expression of at least one of the proposed markers, and/or at least one of the expression markers selected from: IL-6, IL-17A, IL-17F, IL-22 and CCL20 in the biological samples or mixture obtained in b);
d) Selecting drug candidates which are capable of inhibiting the expression or biological function of at least one of the proposed markers, and/or the expression of at least one of the expression markers selected from IL-6, IL-17A, IL-17F, IL-22 and CCL20 measured in said samples or mixtures obtained in b) and comparing the levels with a sample not mixed with the drug candidate (s).

In another embodiment, the invention provides an in vitro screening method of Th17 cells inhibitors for drug candidate identification, comprising the following steps:
a) Collecting at least two biological samples: one mimics the acne lesion, and one mimics the healthy condition;
b) Contacting at least one sample or a mixture of samples with one or more drug candidates to be tested;
c) Detecting the expression of at least one of the proposed markers in the biological samples or mixture obtained in step b);
d) Selecting drug candidates which are capable of inhibiting the expression or biological function of at least one marker chosen from the proposed markers measured in said samples or mixture obtained in step b) and comparing the levels or biological function with a sample not mixed with the drug candidate.

Disclosed herein is also the use of inhibitors identified by screening methods as defined above for the preparation of a composition for treating acne and/or acne associated disorders. More specifically, the disclosure encompasses the use of inhibitors of the proposed markers identified by screening methods for the preparation of a composition for treating acne or acne associated disorders such as N-(2,2,2-trifluoroethyl)-N-[4-[2,2,2-trifluoro-1-hydroxy-1-(trifluoromethyl)ethyl]phenyl]-benzenesulfonamide, 2 oxysterol (oxygenated sterols), especially 24S-hydroxycholesterol 24(S), 25-epoxycholesterol and 7-oxygenated sterols, Methyl 2-cyano-3,12-dioxooleana-1,9(11)dien-28-oate or Bardoxolone methyl, - (8S,9R,10R,13R,14S,16R,17R)-17-[(E,2R)-2, 6-dihydroxy-6-methyl-3-oxohept-4-en-2-yl]-2,16-dihydroxy-4,4,9,13, 14-pentamethyl-8,10,12,15,16, 17-hexahydro-7H-cyclopenta[a]phenanthrene-3,11-dione, 5-(4-chlorophenyl)-6-ethylpyrimidine-2,4-diamine, gamma-D-glutamyl-L-tryptophan, 8-hydroxy-3-methyl-3,4-dihydro-2H-benzo[a]anthracene-1,7,12-trione, 5,7-dihydroxy-2-(4-hydroxyphenyl)-4-oxo-4H-chromen-3-olate, methyl-N-[4-(trifluoromethyl)phenyl]-1,2-oxazole-4-carboxamide or Leflunomide, N-[(E)-(3-methylphenyl)methylideneamino]-6-morpholin-4-yl-2-(2-pyridin-2-ylethoxy)pyrimidin-4-amine.

### Detailed description

The invention is defined by the claims.

Indeed, Th17 cells, a distinct Th lineage originally from the differentiation of naive CD4+ T cell, provide immunity against a variety of extracellular pathogens, including bacteria and fungi. Interestingly, although P.acnes is a commensal bacteria present in healthy human skin, it has been regarded as one of the pathogenetic factors in acne vulgaris. However, it is still not clear whether P. Acnes is indeed a causal agent in the development of non-inflamed and inflamed acne lesions (Shaheen B, Gonzalez M. A microbial aetiology of acne-what is the evidence? Br J Dermatol. 2011 Apr 18).

Th17 cells have also been implicated in a variety of inflammatory and autoimmune disorders, such as psoriasis, rheumatoid arthritis and multiple sclerosis (Peck A, Mellins ED. Precarious balance: Th17 cells in host defense. Infect Immun. 2010 Jan;78(1):32-8).

At molecular level, Th17 cells are characterized by the production of a distinct profile of effector cytokines, IL-17A, IL-17F, IL-26, IL-22, IL-21 and TNF alpha and depend upon IL-23 for their development, survival and proliferation. These cytokines activate different type of cells, such as keratinocytes, leading to their hyperproliferation and further production of proinflammatory cytokines, chemokines and antimicrobial peptides, which in turn recruit and activate other immune cells in the inflamed skin, leading to amplification of the inflammatory response. Moreover, IL-17A, and IL-17F leading to an autocrine regulation of IL-17 production which serves to promote and sustain Th17 cells differentiation (Wei et al. 2007, J Biol. Chem., September 20) . Il 17 is also responsible for the upregulation of CCL20, the ligand of a characterized receptor of the TH17 cells in stromal cells, allowing the attraction of additional Th17 cells into inflamed tissue.

The signalling pathways of the naive CD4 T cell differentiation into Th17 cells required TGFb-1 either in combination with IL-21, with IL-1b and IL-23 or with IL-1b, IL-23, and IL-6, and lead to the expression of retinoid-related orphan receptor (RORC) and retinoid acid-related orphan receptor alpha (RORA), which are two transcription factors that promote TH17 differentiation and substantially upregulate IL-17A and IL-17F expression (Chung Y et al. Critical regulation of early Th17 cell differentiation by interleukin-1 signaling. Immunity 2009;30:576-87, Veldhoen M, Hocking RJ, Atkins CJ, Locksley RM, Stockinger B. and Immunity 2006 Feb; 24 (2) :179-89) .

For the following, "Th-17 differentiation profile molecules" refers to the biological molecules that characterize the Th17 cell differentiation that is to say the cytokines and/or factors of whom depends the differentiation from naive T cell in other words IL-6, IL-26, IL-23 and/or produced by TH17 (IL-17A, IL-17 F, IL-21, IL-22, IL-26, TNF alpha, CCL20), and/or also receptors expressed by TH17 (CCR6, IL-23R).

Interleukin-23 (IL-23) fails to induce the differentiation of naive T cells into Th17 cells but promotes the expansion and survival of Th17 cells. Indeed, IL-23 mediates signalling by binding to a heterodimeric receptor IL-23R/IL-12Rbeta1, and the expression of IL-23 R depends on IL-6, IL-21. (Zhou, L. et al. 2007. IL-6 programs T(H)-17 cell differentiation by promoting sequential engagement of the IL-21 and IL-23 pathways. Nat. Immunol. 8: 967-974. Martinez GJ, et al. Regulation and function of proinflammatory TH17 cells. Ann N Y Acad Sci. 2008 Nov;1143:188-211).

"IL-23R/IL-12Rbeta1" means the heterodimeric receptor comprising IL-23R and IL-12Rbeta1 which is shared by the IL-12 receptor.

Surface phenotype analysis of Th17 cells showed that IL-17A-producing cells express at the same time as IL-23R/IL-12 Rbeta1, the chemokine receptor, CCR6. Interestingly, CCR6 induces homing of cells to skin and plays an etiologic role in many inflammatory diseases considered to be mediated by Th17 cells, including psoriasis, ulcerative colitis, asthma and rheumatoid arthritis (Hedrick MN et al., CCR6 is required for IL-23-induced psoriasis-like inflammation in mice. J Clin Invest. 2009 Aug;119(8):2317-29; Nakae, S. et al. 2003. Suppression of immune induction of collagen-induced arthritis in IL-17-deficient mice. J. Immunol. 171: 6173-6177; Doe C et al. Expression of the T helper 17-associated cytokines IL-17A and IL-17F in asthma and COPD. Chest. 2010 Nov;138 (5) :1140-7; Liu ZJ et al Potential role of Th17 cells in the pathogenesis of inflammatory bowel disease. World J Gastroenterol. 2009 Dec 14;15(46):5784-8)

Different transcription factors are implicated in the described pathway. STAT3 is a critical factor for Th17 differentiation in regulating pathways of IL-17, IL-21, IL-23R and RORC. In particular, in the IL-23 signalling pathway, phosphorylated residues of the intracellular part of IL-23R/IL-12Rbeta1 serve as a docking site for STAT3 which following phosporylation activates the expression of effector cytokines. (Milner,J.D. et al. 2008. Impaired T(H)17 cell differentiation in subjects with autosomal dominant hyper-IgE syndrome. Nature 452: 773-776; Yang XO, et al. (2007) STAT3 regulates cytokine-mediated generation of inflammatory helper T cells. J Biol Chem 282:9358-63.).

Other transcription factors are implicated in the same expression process of the above effector cytokines or RORC such as BATF, IRF4 and AHR (BATF: bringing (in) another Th17-regulating factor. J Mol Cell Biol. 2009 Dec;1(2):66-8).

Therefore, the invention provides the crucial actors of TH17 differentiation as novel markers for acne with the example which follows. The markers for acne according to the invention include (i) at least one of IL-12Rbeta, IL-23R and CCR6 and the corresponding proteins, and (ii) at least one of IL-6, IL-17A, IL17F, IL21, IL-22, IL-26, TNF alpha and CCL20 mRNA and the corresponding protein.

In other words, on the one hand, the disclosure is related to the of the mRNA encoding at least one of the receptors chosen from IL-12Rbeta1/IL-23R, CCR6, and also the corresponding proteins, as markers for acne.

On the other hand, the disclosure is related to the use of the mRNA encoding at least one of the transcription factors chosen from BATF, AHR, STAT3, IRF4, and also the corresponding proteins, as markers for acne.

For the purpose of the present invention, the term "marker" or "biological marker" denotes a biological marker associated with the presence or with the absence of a particular pathological state. The biological markers are in particular proteins, mRNAs.

For more clarity, the following definitions are used: The term "Proposed markers" means IL-12Rbeta1/IL-23R, CCR6 as well as their respective expression product, mRNA or protein. This definition is also applicable to BATF, AHR, STAT 3 and IRF4, respectively.

The term "level of expression" or "expression" means the level of mRNAs or proteins encoded by the gene marker.

The expression level analysis or detection can be performed by any suitable method, known to those skilled in the art, such as western blotting, IHC, mass spectrometry (Maldi-TOF and LC/MS analyses), radioimmunoassay (RIA), Elisa or any other method known to those skilled in the art or else by assaying the mRNA according to the methods customarily known to those skilled in the art. The techniques based on the hybridization of mRNA with specific nucleotide probes are the most customary (Northern blotting, RT-PCR (Reverse Transcriptase Polymerase Chain Reaction), quantitative RT-PCR (qRT-PCR), RNase protection).

The disclosure also provides a method for the diagnosis of acne, comprising the following steps:
a) detecting the level of expression of at least one of the proposed markers and/or at least one of the markers chosen from IL-6, IL-17A, IL-17F, IL-22 and CCL20 in a sample taken from an individual,
b) detecting the level of expression of and at least one of the proposed markers and/or at least one of the markers chosen from IL-6, IL-17A, IL-17F, IL-22 and CCL20 in a sample taken from a healthy individual,
c) comparing the difference in level of expression of at least one marker and for which the level of expression is significantly higher than the level of expression in the healthy individual;
d) the overexpression of at least one of the markers of step c) being an indicator of acne, thus diagnosing acne.

The invention provides also a method for the diagnosis of acne that can also comprise the following steps:
a) detecting the level of expression of at least one of the proposed markers in a sample taken from an individual,
b) detecting the level of expression of at least one of the proposed markers in a sample taken from a normal individual,
c) comparing the difference in level of expression of at least one marker and for which the level of expression is significantly higher than the level of expression in the healthy individual;
d) the overexpression of at least one of the markers of step c) being an indicator of acne, thus diagnosing acne.

The disclosure provides a method for monitoring the progression or variation of acne, comprising the following steps:
a) taking a biological sample from the individual,
b) analysing the level of expression of at least one of the proposed markers, and/or at least one of the markers chosen from IL-6,IL-17A, IL-17F, IL-22, CCL20 in a sample taken and in which a variation in the expression of at least one of the markers is an indicator of the progression of acne. Progression of acne may be from a predominantly comedonal to a more inflammatory dominated state, it may also mean progression towards specific acne subtypes, like nodulocystic acne or acne conglobata for example. Progression might also occur in the other direction, from a more severe to a less severe form of acne.

The disclosure provides also a method for monitoring the efficacy of a treatment intended for treating acne, comprising the following steps:
a) administering the desired treatment to the individual identified as having one or more of the symptoms of acne,
b) taking a biological sample from the individual,
c) analysing the level of expression of at least one of the proposed markers and/or at least one of the other markers chosen from Il-6, IL-17A, IL-17F, IL-22 and CCL20, in which a variation in the expression of at least one of the markers is an indicator of efficacy in the treatment of acne.

The disclosure provides also a in vitro screening method of Th17 cells differentiation inhibitors for treating acne, comprising determining the capacity of said candidate to inhibit or down regulate expression and/or the biological function of one of the proposed markers. More specifically, the disclosure relates to an in vitro screening method of Th-17 cells differentiation inhibitors for the identification of drug candidates, comprising the following steps:
a) Collecting at least two biological samples: one mimics the acne lesion, and one mimics the healthy condition;
b) Contacting at least one sample or a mixture of samples with one or more drug candidates to be tested;
c) Detecting the expression or biological function of at least one of the proposed markers, and/or at least one of the expression markers selected from: IL-6, IL-17 A, IL-17F, IL-22 and CCL20 in the biological samples or mixture obtained in b);
d) Selecting drug candidates which are capable of inhibiting the expression or biological function of at least one of the proposed markers, and/or the expression of at least one of the expression markers selected from IL-6, IL-17A, IL-17F, IL-22 and CCL20 measured in said samples or mixtures obtained in b) and comparing the levels with a sample not mixed with the drug candidate (s).

The expression "overexpression of one of the factors or markers" is intended to mean a level of expression increased by at least 50%, and preferably by at least 100%, and even more preferably by at least 200%, or expressed differently with equivalent significance, by at least a factor of 2, or at least twice as high as the level in a normal individual; which demonstrates overall an overexpression of the chemokines, the cytokines and the receptors mentioned above, thus representing markers characteristic of acne.

In the context of the invention, the biological sample corresponds to any type of sample taken from an individual, and can be a tissue sample or a fluid sample, such as blood, lymph or interstitial fluid.

According to one particular and preferred embodiment, the sample is a biopsy of varying size (preferably from 1 to 6 mm in diameter), or a skin sample taken by means of tape stripping, such as with D-Squames, according to the method described in Wong R et al., "Analysis of RNA recovery and gene expression in the epidermis using non-invasive tape stripping"; J Dermatol Sci.2006 Nov; 44(2):81-92; or in Benson NR, et al., "An analysis of select pathogenic messages in lesional and non-lesional psoriatic skin using non-invasive tape harvesting". J Invest Dermatol. 2006 Oct; 126(10): 2234-41; or else in Wong R et al., "Use of RT-PCR and DNA microarrays to characterize RNA recovered by non-invasive tape harvesting of normal and inflamed skin". J Invest Dermatol. 2004 Jul; 123 (1) : 159-67. According to the principle of tape stripping, the product used comprises a flexible translucent polymer support and an adhesive. The product is applied repeatedly to the skin of the patient, preferably until loss of adhesion. The sample obtained relates only to the content of the outermost layers of the epidermis. A method for analysing a protein content obtained in particular according to this sampling method is described in Patent Application WO2009/068825 (Galderma R&D) in order to monitor markers specific for a pathological skin condition and to orient the diagnosis. Since this method is rapid, non-invasive and relatively inexpensive for detecting the presence of, the absence of or the variation in certain proteomic markers, it is particularly preferred. This method is in particular characterized by mass spectrometry detection, ELISA or any other method known to the expert skilled in the art of protein quantification. Quantification is performed in the skin sample obtained on the flexible and adhesive support in order to detect at least one protein of which the presence, the absence or the variation in amount or in concentration compared with a standard value is associated with the presence, with the progression or with the absence of a particular pathological skin condition.

Another embodiment of the present disclosure is an in vitro screening method of Th17 cell differentiation candidate inhibitors for treating acne, comprising determining the capacity of said candidate to inhibit and/or down regulate the expression or the biological activity or the biological function, including the transactivation properties, of at least one of the proposed markers of the disclosure.

The identified candidate will influence the biological function of a given marker or a biological process modulated by the marker. For example, the inhibition of ROR gamma t and/or ROR alpha by a candidate may affect the biological function of ROR gamma t, including the induction of the Th17 cell differentiation as well as the function of Th17 cells.

For screening purposes, the biological samples consist of transfected cells containing reporter genes operating under the control of a promoter (totally or partially) controlling the expression of an above mentioned gene. Alternatively, the promoter may be, at least in part, synthetically assembled and contain ROR-responsive elements. The ability of a compound to modulate the function of the proposed markers, is evaluated by analyzing the expression of the reporter gene.

The transfected cells may further be engineered to express at least one of the proposed markers.

The reporter gene may encode an enzyme that with its corresponding substrate, provides coloured product(s) such as CAT (chloramphenicol acetyltransferase), GAL (beta galactosidase), or GUS (beta glucuronidase). It might be either luciferase or GFP (Green Fluorescent Protein).

Reporter gene protein dosage or its activity is typically assessed by colourimetric, fluorometric or chemoluminescence methods.

According to a further embodiment of the disclosure, biological samples are cells expressing the gene of interest and the step c) above consists to measure the activity of the gene product.

Further disclosed herein is the use of identified inhibitors/antagonists/inverse agonists with the described screening methods for the preparation of a composition for treating acne and/or acne associated disorders.

In particular, the inhibitors/antagonists/inverse agonists of gamma t or ROR alpha could be selected from the following list:
- N-(2,2,2-trifluoroethyl)-N-[4-[2,2,2-trifluoro-1-hydroxy-1-(trifluoromethyl)ethyl]phenyl]-benzenesulfonamide; this compound is a novel retinoic acid receptor-related orphan receptor-alpha/gamma inverse agonist. (Mol Pharmacol. 2010 Feb;77(2):228-36))
- 2 oxysterol (oxygenated sterols), especially 24S-hydroxycholesterol 24 (S), 25-epoxycholesterol and 7-oxygenated sterols [a second class of nuclear receptors for oxysterols: Regulation of RORalpha and RORgamma activity by 24S-hydroxycholesterol (cerebrosterol)- Wang Y et al. Biochim Biophys Acta. 2010 Aug; 1801(8):917-23. Epub 2010 Mar 6]; Wang Y et al. Modulation of retinoic acid receptor-related orphan receptor alpha and gamma activity by 7-oxygenated sterol ligands. J Biol Chem. 2010 Feb 12; 285(7):5013-25))
- Methyl2-cyano-3,12-dioxooleana-1,9(11)dien-28-oate or Bardoxolone methyl (also known as "RTA 402" and "CDDO-methyl ester).
- (8S,9R,10R,13R,14S,16R,17R)-17-[(E,2R)-2, 6-dihydroxy-6-methyl-3-oxohept-4-en-2-yl]-2,16-dihydroxy-4,4,9,13, 14-pentamethyl-8,10,12,15,16, 17-hexahydro-7H-cyclopenta[a]phenanthrene-3,11-dione or SI-124 (Blaskovich MA, Sun J, Cantor A et al.Discovery of JS-124 (cucurbitacin I), a selective Janus Kinase/ Signal Transducer and Activator of Transcription 2 signaling pathway inhibitor with potent antitumor activity against human and murine cancer cells in mice Cancer Res 2003; 63: 1270-1279)
- Pyrimethamine: - 5-(4-chlorophenyl)-6-ethylpyrimidine-2,4-diamine or Pyrimethamine (Dariprim)(WO/2008/156644)
- gamma-D-glutamyl-L-tryptophan or SCV-07 (SciClone Pharmaceuticals)(Nagabhushanam V, Subbarao K, Ramachandran M et al Inhibition of STAT3 driven gene expression in melanoma cells by SCV-07 J Clin Oncol 2008; 26 (May 20, suppl): 14619)
- 8-hydroxy-3-methyl-3,4-dihydro-2H-benzo[a]anthracene-1,7,12-trione or STA-21 (Song H, Wang R, Wang S et al.A low-molecular-weight compound discovered through virtual database screening inhibits Stat3 function in breast cancer cells PNAS 2005; 102: 4700-4705
- natural flavonol: such as 5,7-dihydroxy-2-(4-hydroxyphenyl)-4-oxo-4H-chromen-3-olate or Kaempferol (Bruno RD, Njar VC. Targeting cytochrome P450 enzymes: a new approach in anticancer drug development. Bioorg Med Chem. 2007 Aug 1;15(15):5047-60. Epub 2007 May 23).
- methyl-N-[4-(trifluoromethyl)phenyl]-1,2-oxazole-4-carboxamide or Leflunomide (O'Donnell EF, Saili KS, Koch DC, Kopparapu PR, Farrer D, Bisson WH, Mathew LK, Sengupta S, Kerkvliet NI, Tanguay RL, Kolluri SK. The anti-inflammatory drug leflunomide is an agonist of the aryl hydrocarbon receptor. PLoS One. 2010 Oct 1;5(10).
- N-[(E)-(3-methylphenyl)methylideneamino]-6-morpholin-4-yl-2-(2-pyridin-2-ylethoxy)pyrimidin-4-amine or STA 5326 (Apilimod Synta pharmaceuticals) Wada et al: Selective abrogation of Th1 response by STA-5326, a potent IL-12/IL-23 inhibitor. Blood, 2007, 109(3), 1156-1164. ; Wada et al: IL-12/IL-23 inhibitors: a promising approach to the treatment of inflammatory disorders. Drugs Fut. 2008, 33(1), 49-63 -[(3S,5R,8R,9S,10S,12R,13S,14S)-3-[(2S,4S,5R,6R)-5-[(2S,4S,5R,6R)-5-[(2S,4S,5R,6R)-4,5-dihydroxy-6-methyl-oxan-2-yl]oxy-4-hydroxy-6-methyl-oxan-2-yl]oxy-4-hydroxy-6-methyl-oxan-2-yl] oxy-12,14-dihydroxy-10,13-dimethyl-1, 2,3,4,5,6,7,8,9,11,12,15,16,17-tetra decahydrocyclopenta[a]phenanthren-17-yl]-5H-furan-2-one or Digoxin and its derivatives.

In another aspect, inhibitors might be either a polypeptide, a DNA or an antisense RNA, an si-RNA or a PNA ("Peptide nucleic acid", i-e with a polypeptidic chain substituted by purine and pyrimidine bases and having a DNA-like structure for hybridization to this latter).

The modulator might be an antibody and preferably a monoclonal antibody. Advantageously, the monoclonal antibody is administered to a patient in a sufficient quantity so as the measure a plasmatic concentration is from about 0.01µg/ml to about 100µg/ml, preferred from about 1µg/ml to about 5µg/ml.

The invention is intended for treating acne. By acne it is understood, all acne forms especially simple acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne and also more largely, acne associated disorders (e.g. hyperseborrhoea).

The example which follows illustrates the invention without limiting the scope thereof.

Table 1: mRNA expression measured by Affymetrix Technology. Analysis of Th17 differentiation profile molecules IL-17A, IL-17F, IL-26, IL-6, CCL20 and proposed markers for acne IL-12Rbeta1/IL-23R, CCR6, BATF, AHR, STAT3,IRF4 as well as IL-5, IL-4 IL-13 typically considered as Th2 cytokines.

Table 2: mRNA expression measured by qRT-PCR (TaqManns low density Array technology) of the expression of Th17 differentiation profile molecules: IL-17A, IL-22, IL-23A, CCL20, IL-6 and three of proposed markers: IL-23R, CCR6, STAT3 and also IL-5, IL-4 IL-13 typically considered as Th2 cytokines:
Table 3: Protein expression by Luminex assay of Th17 differentiation profile molecules: IL-6, IL-17A, IL-17F, IL-21, IL-22, IL-23A, CCL20, TNF alpha and as well as IL-5, IL-4 IL-13 typically considered as Th2 cytokines:
FIGURE 1: Acne lesion: T lymphocyte immunohistochemical detection (CD3 alone)
FIGURE 2 : Acne lesion : IL-17 expression in T lymphocytes (CD3/IL-17 co-localisation)

### Example 1: Modulation of the TH17 molecular profile in the lesional skin of patients suffering from acne compared with non-lesional skin of these patients: Analysis of the expression of IL17A, IL17F, IL26 and CCL20 and proposed markers IL-12Rbetal/IL-23R, CCR6, BATF, AHR, STAT3 and IRF4.

### Patient selection and tissue biopsies:

Skin biopsies of acne patients were obtained from an inflammatory papule and from non lesional skin in 12 patients with acne, in accordance with good clinical practice. (The clinical description of acne subtypes was carried out according to the classification of Wilkin et al., 2002, J. Am. Acad. Dermatol. Vol 46, pages 584-587.)

To evaluate a change in the expression level of the genes, the expression levels in lesional skin are compared with the expression levels in non-lesional skin of the same subjects (n=12).

### mRNA extraction, labelling and hybridization to probe arrays :

The mRNA was isolated from skin using the RNeasy extraction kit (Quigen Inc., Valencia,CA) and quality was evaluated using a 2100 Bioanalyser of Agilent. The mRNA expression was evaluated by a Gene Chip IVT labelling kit after the generation of double-stranded cDNA (i.e in vitro transcription process) using T7-oligo primer and the one cycle cDNA synthesis kit of Affymetrix. RNA was ethanol precipitated to concentrate the sample and then quantified using a spectrophotometer. Approximately 200 ng of total RNA of good quality [RNA indication number (RIN) ≥ 7] from each sample was used to generate double-stranded cDNA using a T7-oligo (dt) primer (one cycle cDNA synthesis kit, Affymetrix). Biotinylated cRNA, produced through in vitro transcription (Gene Chip IVT labelling kit, Affymetrix) was fragmented and hybridised to an Affymetrix human U133A 2.0 plus microarray. The arrays were processed on a Gene Chip Fluidics Station 450 and scanned on an Affymetrix Gene Chip Scanner (Santa Clara, CA) .

### Statistical Analysis of mRNA expression based on Affymetrix gene chips:

The expression data from Affymetrix Gene Chips are normalized with RMA (Robust Multi-array Analysis) method. The raw intensity values are background corrected, log2 transformed and then quantile normalized. Next a linear model is fit to the normalized data to obtain an expression measure for each probe set on each array. To identify genes that were significantly modulated in the different Acne subtype samples, one-way ANOVA with Benjamini-Hochberg multiplicity correction was performed using JMP 7.0.1 (SAS Institute) and irMF 3.5 (National Institute of Statistical Sciences, NISS) software.

### qRT-PCR Measurement of mRNA expression:

The expression of the Th17 differentiation profile was also measured by qRT-PCR.

In the following table the expression levels are documented using the Mean Ct (Cycle Threshold) of individual genes in non lesional skin and in lesional skin of acne patients. The Ct value is inversely proportional to the quantity of the mRNA of a given gene.

### Cytokine extraction and assay:

Proteins were extracted from inflammatory papules and non lesional skin in 12 patients with acne. Cytokines were dosed in the protein extracts using Luminex assays (Millipore & Procarta cytokine dosage kits). The cytokine quantities were normalized to the total concentration of protein. Paired P-values were calculated for each cytokine.

The mRNA expression of the Th17 differentiation profile molecules: IL-17A, IL-17F, IL-26, CCL20 and proposed markers IL12Rbeta1/il23R, CCR6, BATF, AHR, STAT3, and IRF4 were measured using Affymetrix technology (Table 1) and qRT-PCR (Table 2).

According to Table 1 and table 2, the mRNA of specific cytokines IL-17A, IL-17F, IL-22, IL-26 characterizing Th17 cells differentiation measured by Affymetrix technique (table 1) or qRT-PCR (table 2) techniques are significantly upregulated in patients with acne. Moreover, in these tables, the mRNA expression of 15, I4 and 113 are not detected or not changed suggesting that the inflammatory response in acne is not driven by Th2 cells.

Table 3 demonstrates an up-regulation of the protein expression level of IL-6, IL-17A, IL-17F, IL-21, IL-22 and TNF alpha in lesional skin in comparison to non-lesional skin.

Surprisingly, the mRNA levels of transcription factors including IL-12Rbeta1/IL-23R, CCR6, BATF, AHR, STAT3 and IRF4 are not modulated in acne, but their expression in human skin was clearly demonstrated. Thus, they are interesting as markers for diagnosing acne and/or screening inhibitors of Th-17 cells differentiation in using them alone or in combination of themselves or with at least one of the Th17 cells differentiation profile molecules as mentioned previously. In particular, the mRNA and protein expression of the CCR6 ligand, CCL20 and the mRNA expression of IL-23A is upregulated in patients with acne and therefore confirms the potential interest of inhibiting its binding with their receptors CCR6 and IL-23Rbeta/IL-23R via a compound and the use these receptors as a markers for acne.

### EXAMPLE 2: Immunohistochemistry analysis:

In normal skin, we observed hardly any lymphocyte infiltrates whereas in biopsies from lesional areas there were found in greater numbers. In this context, IL-17 detection using an immunohistochemistry technique was performed in these infiltrates from lesional areas.

A first primary antibody (anti CD3) was used in order to detect the T lymphocytes, followed by a second antibody, specific for IL-17.

These antibodies were respectively revealed with a second antibody combined with a red fluorophore (TRITC) or a green fluorophore (FITC).

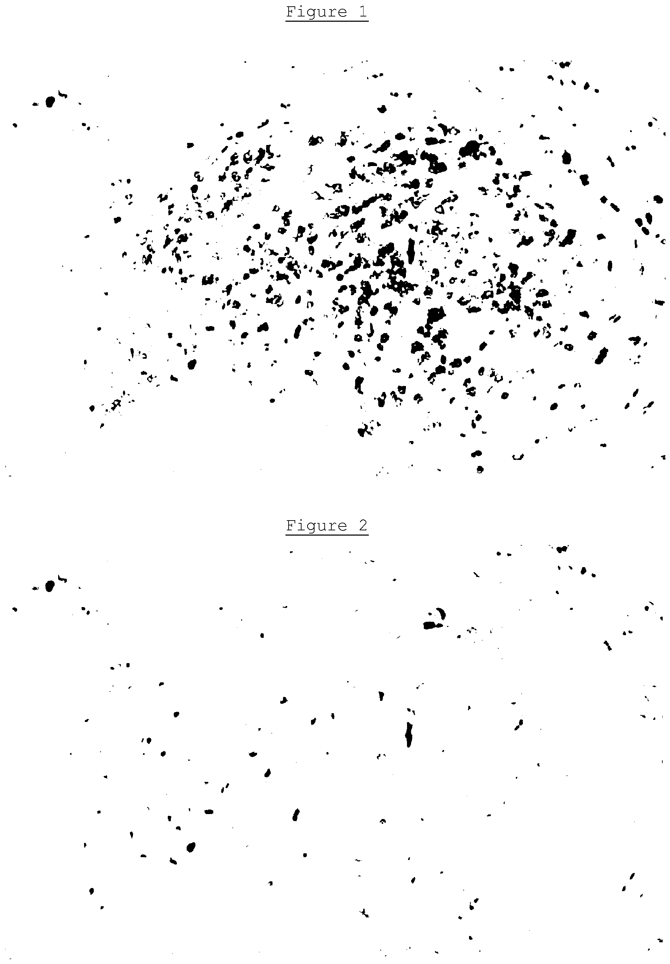

The results for CD3 expression are presented in Figure 1. The positive cells, in black, confirmed that the infiltrate was largely composed of T lymphocytes.

Figure 2 demonstrates that a subpopulation of CD3 positive T lymphocytes co-expressed IL-17. This positive IL-17 staining suggests the presence of Th17 cells in acne lesions.

## Claims

1. Use of (i) the mRNA encoding IL-12Rbeta 1, IL-23R or CCR6, or the corresponding protein, and (ii) the mRNA encoding at least one marker selected from IL-6, IL-17A, IL-17F, IL-21, IL-22, IL-26, TNF alpha and CCL20, or the corresponding protein, as markers for acne.

2. A method for the diagnosis of acne, comprising the following steps of:
a) detecting the level of expression of (i) the mRNA encoding IL-12Rbeta 1, IL-23R or CCR6, or the corresponding protein, and (ii) the mRNA encoding at least one of the markers selected from IL-6, IL-17A, IL-17F, IL-21, IL-22, IL-26, TNF alpha, and CCL20, or the corresponding protein, in a sample from an individual,
b) detecting the level of expression of (i) the mRNA encoding IL-12Rbeta 1, IL-23R or CCR6, or the corresponding protein, and (ii) the mRNA encoding at least one of the markers selected from IL-6, IL-17A, IL-17F, IL-21, IL-22, IL-26, TNF alpha, and CCL20, or the corresponding protein, in a sample from a healthy individual,
c) comparing the levels of expression of the mRNA or the corresponding protein detected in the healthy individual of step b) and in the individual of step a), the overexpression of the mRNA or the corresponding protein, in the individual of step a) being an indicator of acne, thus diagnosing acne.

3. A method for monitoring the progression of acne in an individual, comprising the step of analysing the level of expression of (i) the mRNA encoding IL-12Rbeta 1, IL-23R or CCR6, or the corresponding protein, and (ii) the mRNA encoding at least one distinct marker selected from IL-6, IL-17A, IL-17F, IL-21, IL-22, IL-26, TNF alpha, and CCL20, or the corresponding protein, in a biological sample wherein a variation in the levels of expression of the mRNA or the corresponding protein, at different time is an indicator of the progression of acne.

4. A method for monitoring the efficacy of a treatment for treating acne in an individual, comprising the step of analysing the level of expression of (i) the mRNA encoding IL-12Rbeta 1, IL-23R or CCR6, or the corresponding protein, and (ii) the mRNA encoding at least one distinct marker selected from IL-6, IL-17A, IL-17F, IL-21, IL-22, IL-26, TNF alpha, and CCL20, or the corresponding protein, in a biological sample obtained from an individual identified as having one or more of the symptoms of acne, wherein a variation in the levels of expression of the mRNA or the corresponding protein, before and after administration to the individual of a treatment of acne is an indicator of efficacy in the treatment of acne.

5. An *in vitro* screening method of Th17 cells differentiation inhibitors for treating acne, comprising determining the capacity of a candidate to inhibit or down regulate expression of (i) mRNA encoding IL-12Rbeta 1, IL-23R or CCR6, or the corresponding protein and (ii) the mRNA encoding at least one marker selected from IL-6, IL-17A, IL-17F, IL-21, IL-22, IL-26, TNF alpha and CCL20, or the corresponding protein.

6. An *in vitro* screening method of Th17 cells differentiation inhibitors for the identification of drug candidates for treating acne, comprising the following steps of:
a) contacting a least one biological sample mimicking an acne lesion or a mixture of biological samples mimicking an acne lesion with one or more drug candidates to be tested;
b) detecting the expression of (i) the mRNA encoding IL-12Rbeta 1, IL-23R or CCR6, or the corresponding protein, and (ii) the mRNA encoding at least one distinct marker selected from IL-6, IL-17A, IL-17F, IL-21, IL-22, IL-26, TNF alpha, and CCL20, or the corresponding protein, in the biological sample or mixture of biological samples of step a);
c) comparing the expression detected in step b) to the expression detected in a sample mimicking an acne lesion which has not been contacted with a drug candidate; and
d) selecting drug candidates which inhibit the expression of (i) the mRNA encoding IL-12Rbeta 1, IL-23R or CCR6, or the corresponding protein, and (ii) the mRNA encoding at least one distinct marker selected from IL-6, IL-17A, IL-17F, IL-21, IL-22, IL-26, TNF alpha, and CCL20, or the corresponding protein, in the biological sample or mixture of biological samples of step a), as detected in step b), said selected candidates being identified as the Th17 cells differentiation inhibitors.
**Table 1**
| GENE_SYMBOL | TITLE | Non lesional skin Mean_Expressions | Lesional skin Mean_Expressions | Lesional skin vs non lesional skin Fold Change | Lesional skin vs non lesional skin Adjusted Pvalue |
|---|---|---|---|---|---|
| IL6 | interleukin 6 (interferon, beta 2) | 73 | 340 | 4,7 | 1,3E-03 |
| IL17A | interleukin 17A | 13 | 57 | 4,3 | 7,8E-03 |
| CCL20 | chemokine (C-C motif) ligand 20 | 146 | 482 | 3,3 | 1,6E-03 |
| IL17F | interleukin 17F | 35 | 88 | 2,5 | 1.1E-02 |
| IL26 | interleukin 26 | 24 | 48 | 2,0 | 2,3E-03 |
| STAT3 | signal transducer and activator of transcription 3 (acute-phase response factor) | 962 | 1853 | 1,9 | 2.0E-04 |
| | | | | | |
| BATF | basic leucine zipper transcription factor, ATF-like | 163 | 309 | 1,9 | 1,2E-03 |
| IL12RB1 | interleukin 12 receptor, beta 1 | 73 | 115 | 1,6 | 1,7E-03 |
| CCR6 | chemokine (C-C motif) receptor 6 | 91 | 106 | 1,2 | 3,3E-01 |
| IRF4 | interferon regulatory factor 4 | 214 | 230 | 1,1 | 3.0E-01 |
| AHR | aryl hydrocarbon receptor | 1712 | 1738 | 1,0 | 7.7E-01 |
| IL23R | interleukin 23 receptor | not detected | not detected | | |
| IL5 | interleukin 5 (colony-stimulating factor, eosinophil) | Not detected | Not detected | | |
| IL4 | interleukin 4 | Not detected | Not detected | | |
| IL13 | inlerleukin 13 | 78,61 | 79,65 | 1,0 | 8,0E-01 |
**Table 2**
| GENE_SYMBOL | TITLE | Non lesional skin Mean_CT | Lesional skin Mean_CT | Lesional skin vs non lesional skin Fold Change | Lesional skin vs non lesional skin P-value |
|---|---|---|---|---|---|
| I123A | interleukin 23, alpha subunit p19 | 32,8 | 30,1 | 6,23 | 1,5E-08 |
| IL6 | interleukin 6 (*interferon, beta* 2) | 30,3 | 27,7 | 6,22 | <0.0001 |
| IL17A | interleukin 17A | 32,3 | 29,9 | 5,55 | <0.0001 |
| IL22 | interleukin 22 | 34,0 | 32,7 | 2,42 | 8,1E-06 |
| CCL20 | chemokine (C-C motif) ligand 20 | 29,2 | 28,0 | 2,35 | 6,8E-10 |
| IL23R | interleukin 23 receptor | 34,3 | 34,6 | -1,2 | 1.0E-01 |
| STAT3 | signal transducer and activator of transcription 3 (acute-phase response factor) | 22,1 | 22,0 | -1,39 | 2,SE-01 |
| CCR6 | chemokine (C-C motif) receptor 6 | 29,1 | 28,2 | -1,75 | 9.8E-11 |
| IL5 | interleukin 5 (colony-stimulating factor, eosinophil) | 34,3 | 35,4 | -2,08 | 5,26E-13 |
| IL4 | interleukin 4 | 34,2 | 35,3 | -2,12 | 2,03E.08 |
| IL13 | interleukin 13 | 34,3 | 35,4 | -2,19 | 2,53E-13 |
**Table 3**
| Proteins | LSP | NSP | FoldChange | PValue |
|---|---|---|---|---|
| Interleukin-6 | 16,2 | 1,1 | 15,0 | 0,0005 |
| interleukin-17F | 1,2 | 0,3 | 4,9 | 3,2E-03 |
| Interleukin-21 | 29,1 | 7,5 | 3,9 | 1,1E-02 |
| Interleukin-17A | 4,0 | 1,1 | 3,8 | 0,0081 |
| Interleukin-22 | 14,3 | 4,6 | 3,1 | 3,2E-02 |
| Interleukin-23 subunit alpha | 1,0 | 0,3 | 3,1 | 1,7E-01 |
| C-C motif chemokine 20 | 1,0 | 0,4 | 2,6 | 4,0E-02 |
| Tumor necrosis factor alpha | 2,6 | 1,1 | 2,5 | 0,0182 |
| Interleukin-4 | 4,2 | 1,9 | 2,2 | 0,0052 |
| Interleukin-5 | not detected | not detected | | |
| Interleukin-13 | 3,3 | 2,1 | 1,6 | 0,0678 |

## Patentansprüche

1. Verwendung von (i) der mRNA, die für IL-12Rbeta 1, IL-23R oder CCR6 kodiert, oder dem entsprechenden Protein, und (ii) der mRNA, die zumindest für einen Marker kodiert, der aus IL-6, IL-17A, IL-17F, IL-21, IL-22, IL-26, Tumornekrosefaktor-α bzw. TNF-α und CCL20 ausgewählt ist, oder dem entsprechenden Protein, als Marker für Akne.

2. Verfahren zur Diagnose von Akne, das folgende Schritte aufweist:
(a) Detektieren des Expressionsniveaus (i) der mRNA, die für IL-12Rbeta 1, IL-23R oder CCR6 kodiert, oder des entsprechenden Proteins, und (ii) der mRNA, die für zumindest einen der Marker kodiert, der aus IL-6, IL-17A, IL-17F, IL-21, IL-22, IL-26, TNF-α und CCL20 ausgewählt ist, oder des entsprechenden Proteins, in einer Probe eines Individuums,
(b) Detektieren des Expressionsniveaus (i) der mRNA, die für IL-12Rbeta 1, IL-23R oder CCR6 kodiert, oder des entsprechenden Proteins, und (ii) der mRNA, die für zumindest einen der Marker kodiert, der aus IL-6, IL-17A, IL-17F, IL-21, IL-22, IL-26, TNF-α und CCL20 ausgewählt ist, oder des entsprechenden Proteins, in einer Probe eines gesunden Individuums,
(c) Vergleichen der Expressionsniveaus der mRNA oder des entsprechenden Proteins, das in dem gesunden Individuum von Schritt b) und in dem Individuum von Schritt a) detektiert wurde, wobei die Überexpression der mRNA oder des entsprechenden Proteins in dem Individuum von Schritt a) ein Indikator für Akne ist, wodurch Akne diagnostiziert wird.

3. Verfahren zum Überwachen der Progression bzw. des Fortschreitens von Akne in einem Individuum, welches den Schritt des Analysierens des Expressionsniveaus (i) der mRNA, die für IL-12Rbeta 1, IL-23R oder CCR6 kodiert, oder des entsprechenden Proteins, und (ii) der mRNA, die für zumindest einen bestimmten Marker kodiert, der aus IL-6, IL-17A, IL-17F, IL-21, IL-22, IL-26, TNF-α und CCL20 ausgewählt ist, oder des entsprechenden Proteins, in einer biologischen Probe, wobei eine Veränderung des Expressionsniveaus der mRNA oder des entsprechenden Proteins, zu unterschiedlichen Zeiten ein Indikator für das Fortschreiten von Akne ist.

4. Verfahren zum Überwachen der Wirksamkeit einer Behandlung zum Behandeln von Akne bei einem Individuum, das den folgenden Schritt aufweist: Analysieren des Expressionsniveaus (i) der mRNA, die für IL-12Rbeta 1, IL-23R oder CCR6 kodiert, oder des entsprechenden Proteins, und (ii) der mRNA, die zumindest für einen bestimmten Marker kodiert, der aus IL-6, IL-17A, IL-17F, IL-21, IL-22, IL-26, TNF-α und CCL20 ausgewählt ist, oder des entsprechenden Proteins, in einer biologischen Probe, die einem Individuum entnommen wurde, bei dem festgestellt wurde, dass es ein oder mehrere Symptome von Akne hat, wobei eine Veränderung des Expressionsniveaus der mRNA oder des entsprechenden Proteins vor und nach der Durchführung einer Aknebehandlung an dem Individuum ein Indikator der Wirksamkeit für die Behandlung von Akne ist.

5. In vitro Screening-Verfahren für Th17 Zelldifferenzierungsinhibitoren zur Behandlung von Akne, welches das Bestimmen der Kapazität eines Kandidaten aufweist, die Expression (i) der mRNA, die für IL-12Rbeta 1, IL-23R oder CCR6 kodiert, oder des entsprechenden Proteins, und (ii) der mRNA, die zumindest für einen Marker kodiert, der aus IL-6, IL-17A, IL-17F, IL-21, IL-22, IL-26, TNF-α und CCL20 ausgewählt ist, oder des entsprechenden Proteins, zu hemmen oder runter zu regulieren.

6. In vitro Screening-Verfahren für Th17 Zelldifferenzierungsinhibitoren zur Identifikation von Wirkstoffkandidaten zum Behandeln von Akne, welches folgende Schritte aufweist:
(a) In-Kontakt-Bringen mindestens einer biologischen Probe, welche eine Akne-Läsionen imitiert, oder einer Mischung von biologischen Proben, die eine Akne-Läsion imitiert, mit einem oder mehreren Wirkstoffkandidaten, die getestet werden sollen;
b) Detektieren der Expression (i) der mRNA, die für IL-12Rbeta 1, IL-23R oder CCR6 kodiert, oder des entsprechenden Proteins, und (ii) der mRNA, die zumindest für einen bestimmten Marker kodiert, der aus IL-6, IL-17A, IL-17F, IL-21, IL-22, IL-26, TNF-α und CCL20 ausgewählt ist, oder des entsprechenden Proteins, in der biologischen Probe oder Mischung von biologischen Proben des Schrittes a),
(c) Vergleichen der Expression, die in Schritt b) detektiert ist, mit der Expression, die in einer Probe detektiert ist, die eine Akne-Läsion imitiert, welche nicht mit einem Wirkstoffkandidaten in Kontakt gebracht worden ist; und
(d) Auswählen von Wirkstoff-Kandidaten, welche die Expression (i) der mRNA, die für IL-12Rbeta 1, IL-23R oder CCR6 kodiert, oder des entsprechenden Proteins, und (ii) der mRNA, die zumindest für einen bestimmten Marker kodiert, der aus IL-6, IL-17A, IL-17F, IL-21, IL-22, IL-26, TNF-α und CCL20 ausgewählt ist, oder des entsprechenden Proteins, in der biologischen Probe oder der Mischung von biologischen Proben von Schritt a) hemmen, wie im Schritt b) detektiert, wobei die ausgewählten Kandidaten als die Th17 Zelldifferenzierungsinhibitoren identifiziert werden.

## Revendications

1. Utilisation (i) de l'ARNm codant pour l'IL-12R bêta 1, l'IL-23R ou le CCR6, ou de la protéine correspondante, et (ii) de l'ARNm codant pour au moins un marqueur choisi parmi l'IL-6, l'IL-17A, l'IL-17F, l'IL-21, l'IL-22, l'IL-26, le TNF alpha et le CCL20 ou de la protéine correspondante, en tant que marqueurs de l'acné.

2. Procédé de diagnostic de l'acné, comprenant les étapes suivantes :
a) détecter le niveau d'expression (i) de l'ARNm codant pour l'IL-12R bêta 1, l'IL-23R ou le CCR6 ou de la protéine correspondante, et (ii) de l'ARNm codant pour au moins l'un des marqueurs choisi parmi l'IL-6, l'IL-17A, l'IL-17F, l'IL-21, l'IL-22, l'IL-26, le TNF alpha et le CCL20 ou de la protéine correspondante, dans un échantillon en provenance d'un individu,
b) détecter le niveau d'expression (i) de l'ARNm codant pour l'IL-12R bêta 1, l'IL-23R ou le CCR6 ou de la protéine correspondante, et (ii) de l'ARNm codant pour au moins l'un des marqueurs choisi parmi l'IL-6, l'IL-17A, l'IL-17F, l'IL-21, l'IL-22, l'IL-26, le TNF alpha et le CCL20 ou de la protéine correspondante, dans un échantillon en provenance d'un individu sain,
c) comparer les niveaux d'expression de l'ARNm ou de la protéine correspondante détectés chez l'individu sain de l'étape b) et chez l'individu de l'étape a), la surexpression de l'ARNm ou de la protéine correspondante, chez l'individu de l'étape a) étant un indicateur d'acné, permettant ainsi le diagnostic de l'acné.

3. Procédé de surveillance de la progression de l'acné chez un individu, comprenant l'étape d'analyse du niveau d'expression (i) de l'ARNm codant pour l'IL-12R bêta 1, l'IL-23R ou le CCR6 ou de la protéine correspondante, et (ii) de l'ARNm codant pour au moins un marqueur distinct choisi parmi l'IL-6, l'IL-17A, l'IL-17F, l'IL-21, l'IL-22, l'IL-26, le TNF alpha et le CCL20 ou de la protéine correspondante, dans un échantillon biologique, dans lequel une variation des niveaux d'expression de l'ARNm ou de la protéine correspondante, à des instants différents est un indicateur de la progression de l'acné.

4. Procédé de surveillance de l'efficacité d'un traitement destiné à traiter l'acné chez un individu, comprenant l'étape d'analyse du niveau d'expression (i) de l'ARNm codant pour l'IL-12Rbêta 1, l'IL-23R ou le CCR6 ou de la protéine correspondante, et (ii) de l'ARNm codant pour au moins un marqueur distinct choisi parmi l'IL-6, l'IL-17A, l'IL-17F, l'IL-21, l'IL-22, l'IL-26, le TNF alpha et le CCL20 ou de la protéine correspondante, dans un échantillon biologique obtenu en provenance d'un individu identifié comme présentant un ou plusieurs des symptômes de l'acné, dans lequel une variation des niveaux d'expression de l'ARNm ou de la protéine correspondante, avant et après l'administration à l'individu d'un traitement de l'acné est un indicateur de l'efficacité du traitement de l'acné.

5. Procédé de dépistage *in vitro* d'inhibiteurs de différentiation des cellules Th17 pour le traitement de l'acné, comprenant la détermination de la capacité d'un candidat à inhiber ou à réguler à la baisse l'expression (i) de l'ARNm codant pour l'IL-12R bêta 1, l'IL-23R ou le CCR6 ou de la protéine correspondante, et (ii) de l'ARNm codant pour au moins un marqueur choisi parmi l'IL-6, l'IL-17A, l'IL-17F, l'IL-21, l'IL-22, l'IL-26, le TNF alpha et le CCL20 ou de la protéine correspondante.

6. Procédé de dépistage *in vitro* d'inhibiteurs de différentiation des cellules Th17 pour l'identification de médicaments candidats pour le traitement de l'acné, comprenant les étapes suivantes :
a) mettre en contact au moins un échantillon biologique imitant une lésion acnéique ou un mélange d'échantillons biologiques imitant une lésion acnéique avec un ou plusieurs médicaments candidats à tester ;
b) détecter l'expression (i) de l'ARNm codant pour l'IL-12R bêta 1, l'IL-23R ou le CCR6 ou de la protéine correspondante, et (ii) de l'ARNm codant pour au moins un marqueur distinct choisi parmi l'IL-6, l'IL-17A, l'IL-17F, l'IL-21, l'IL-22, l'IL-26, le TNF alpha et le CCL20 ou de la protéine correspondante, dans l'échantillon biologique ou le mélange d'échantillons biologiques de l'étape a) ;
c) comparer l'expression détectée à l'étape b) à l'expression détectée dans un échantillon imitant une lésion acnéique n'ayant pas été en contact avec un médicament candidat ; et
d) sélectionner les médicaments candidats qui inhibent l'expression (i) de l'ARNm codant pour l'IL-12R bêta 1, l'IL-23R ou le CCR6 ou de la protéine correspondante, et (ii) de l'ARNm codant pour au moins un marqueur distinct choisi parmi l'IL-6, l'IL-17A, l'IL-17F, l'IL-21, l'IL-22, l'IL-26, le TNF alpha et le CCL20 ou de la protéine correspondante, dans l'échantillon biologique ou le mélange d'échantillons biologiques de l'étape a), telle que détectée à l'étape b), lesdits candidats sélectionnés étant identifiés comme inhibiteurs de différentiation des cellules Th17.
